# FASCICULE DE BREVET EUROPEEN

(11) **EP 2 839 775 B1**
(45) Date de publication et mention de la délivrance du brevet: **26.04.2017**
(21) Numéro de dépôt: 14180922.8
(22) Date de dépôt: 14.08.2014
(51) Int. Cl.: A61B 5/00, A61B 5/11

(54) **Dispositif de détermination d'une position relative d'un fémur par rapport à un tibia**
Verrichtung zur relativen Positionsbestimmung eines Femurs im Verhältnis zu einem Schienbein
Device for determining a relative position of a femur in relation to a tibia

(30) Priorité: 19.08.2013 FR 1358048
(43) Date de publication de la demande: 25.02.2015
(73) Titulaire: Amplitude, 26000 Valence (FR)
(72) Inventeur: Jallabert, Olivier, 26000 Valence (FR); Geais, Laurent, 26100 Romans (FR); Jugnet, Bruno, 69004 Lyon (FR)
(74) Mandataire: Chevalier, Renaud Philippe

(56) Documents cités:
- WO-A1-01/32080
- WO-A1-02/18019
- HAMIDREZA BAYATI ET AL: "Unsupervised Adaptation to On-body Sensor Displacement in Acceleration-Based Activity Recognition", WEARABLE COMPUTERS (ISWC), 2011 15TH ANNUAL INTERNATIONAL SYMPOSIUM ON, IEEE, 12 juin 2011 (2011-06-12), pages 71-78, XP031903412, DOI: 10.1109/ISWC.2011.11 ISBN: 978-1-4577-0774-2
- IVANA MILOVANOVIC: "Radial Basis Function (RBF) networks for improved gait analysis", NEURAL NETWORK APPLICATIONS IN ELECTRICAL ENGINEERING, 2008. NEUREL 2008. 9TH SYMPOSIUM ON, IEEE, PISCATAWAY, NJ, USA, 25 septembre 2008 (2008-09-25), pages 129-132, XP031365268, ISBN: 978-1-4244-2903-5
- MOTE C D ET AL: "Identification of knee joint models for varus-valgus and internal-external rotations: Snow skiing experiments", JOURNAL OF BIOMECHANICS, PERGAMON PRESS, NEW YORK, NY, US, vol. 22, no. 3, 1 janvier 1989 (1989-01-01), pages 245-249,251, XP026270785, ISSN: 0021-9290, DOI: 10.1016/0021-9290(89)90092-4 [extrait le 1989-01-01]

## Description

L'invention concerne un dispositif de détermination d'une position relative d'un fémur par rapport à un tibia.

### ARRIERE PLAN TECHNOLOGIQUE DE L'INVENTION

Une pose d'une prothèse totale de genou vise à restaurer une articulation entre un fémur et un tibia d'un patient. Ladite prothèse doit reproduire le plus précisément possible le genou qu'elle remplace pour être portée sans douleur ni gêne par le patient.

Il s'avère que le genou est une articulation particulièrement complexe. Il est donc primordial de correctement étudier le genou pour pouvoir construire une prothèse la plus proche possible du genou natif.

A cet effet, il est connu d'étudier une morphologie des surfaces externes d'un tibia et d'un fémur associés au genou étudié. Différentes mesures géométriques sont également réalisées comme le relevé d'un ou plusieurs ratios Antéro-Postérieur/ Médio-Latéral d'une extrémité distale du fémur ou du tibia.

Il est également connu du document EP2042110A1, un dispositif se présentant sous la forme d'une genouillère destinée à être enfilée sur un genou. Le dispositif est conformé de sorte que lorsque la genouillère est en place sur le genou, un premier capteur est disposé au niveau du fémur et un deuxième capteur est disposé au niveau du tibia. Chaque capteur délivre une information de sa position et un organe de traitement traite ces informations pour en déduire des données concernant l'angle formé entre le tibia et le fémur.

Dans ledit document, il est considéré que le fémur et le tibia forme une liaison parfaite pour déterminer des données concernant l'angle formé entre le tibia et le fémur. Or ceci est une très grosse approximation.

Le document WO 02/18012 A1 décrit la détermination d'un angle entre la cuisse et la jambe d'un sujet utilisant un gyroscope attaché à la cuisse et un gyroscope attaché à la jambe.

### OBJET DE L'INVENTION

Un but de l'invention est de proposer un dispositif permettant d'étudier plus précisément un genou en déterminant une position relative d'un fémur par rapport à un tibia, le fémur et le tibia étant associés audit genou.

### BREVE DESCRIPTION DE L'INVENTION

En vue de la réalisation de ce but, on propose un dispositif de détermination d'une position relative d'un fémur par rapport à un tibia comportant au moins :
- un premier organe de mesure destiné à être positionné contre le tibia et comportant des moyens pour générer un signal représentatif d'une position de l'organe de mesure ;
- un deuxième et un troisième organe de mesure destinés à être positionnés relativement au fémur en encerclant chacun la cuisse associée au fémur à des hauteurs différentes, le deuxième et le troisième organe de mesure comportant chacun des moyens pour générer un signal représentatif d'une position de l'organe de mesure associé ;
- un organe de calcul comportant des moyens de communication pour recevoir au moins les signaux générés par le premier, deuxième et troisième organes de mesure, des moyens de comparaison qui confrontent les signaux des deuxième et troisième organes pour en déduire une position absolue du fémur et des moyens de traitement qui analysent le signal du premier organe de mesure et la position absolue du fémur pour en déduire une position relative du fémur par rapport au tibia.

La détermination d'une position absolue d'un tibia s'avère simple. En effet, le tibia est directement accessible à travers une fine épaisseur de peau, de sorte que le placement du premier organe de mesure contre le tibia donne accès à la position absolue de celui-ci. En revanche, il n'est pas possible de réaliser un contact direct entre un capteur et un fémur ce qui rend la détermination de la position du fémur bien plus difficile. Les inventeurs ont ainsi eu l'idée de disposer des organes de mesures à différentes hauteurs de la cuisse pour croiser les mesures réalisées et ainsi estimer une position absolue du fémur.

Ainsi, le dispositif de l'invention permet de repérer une position relative du tibia par rapport au fémur. Le dispositif permet d'étudier bien plus précisément une articulation entre un fémur et un tibia qu'un dispositif de l'art antérieur.

Selon un mode de réalisation de l'invention, le deuxième et le troisième organes de mesure comportent chacun des moyens pour relever des données représentatives d'une position du fémur.

Selon un mode de réalisation de l'invention, les moyens pour relever des données représentatives d'une position du fémur comportent un premier capteur de position et un deuxième capteur de position, chaque capteur comportant deux accéléromètres.

Selon un mode de réalisation de l'invention, le dispositif de détermination est conformé de sorte que les capteurs soient disposés à des positions éloignées sur une même hauteur de cuisse.

Selon un mode de réalisation de l'invention, le dispositif de détermination comporte en outre un quatrième organe de mesure destiné à être positionné relativement au fémur en encerclant la cuisse associée au fémur à une hauteur différente du deuxième et du troisième organe de mesure, le quatrième organe comportant des moyens pour relever des données représentatives d'une position du fémur et des moyens pour générer un signal représentatif de ces données.

### BREVE DESCRIPTION DES DESSINS

L'invention sera mieux comprise à la lumière de la description qui suit d'un mode de réalisation particulier non limitatif de l'invention en référence aux figures ci-jointes parmi lesquelles :
- la figure 1 est une vue schématique d'un dispositif de détermination selon l'invention disposé en partie sur un membre inférieur d'un patient;
- la figure 2 est une vue schématique d'un organe de calcul et d'un premier organe de mesure du dispositif illustré à la figure 1 ;
- la figure 3 est une vue schématique d'un organe de calcul ainsi que d'un deuxième, troisième et quatrième organe de mesure du dispositif illustré à la figure 1, lesdits organes de mesure étant disposés en partie sur un membre inférieur du patient.

### DESCRIPTION DETAILLEE DE L'INVENTION

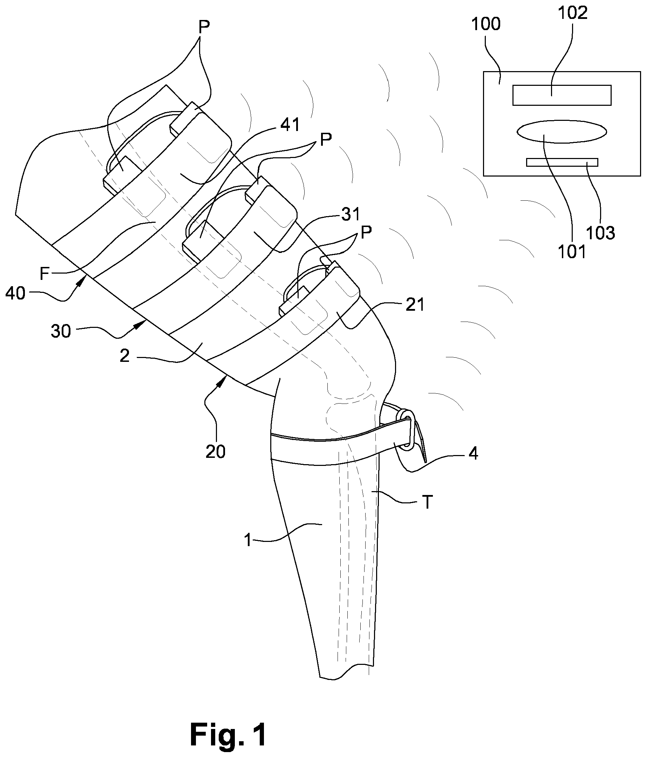

En référence à la figure 1, le dispositif de détermination selon l'invention permet d'étudier une position relative d'un fémur F par rapport à un tibia T.

Le dispositif comporte un premier organe de mesure 4 destiné à être positionné contre le tibia T. Le premier organe de mesure 4 est conformé pour épouser au mieux la morphologie du tibia T et avoir ainsi une surface de contact optimale avec le tibia T.

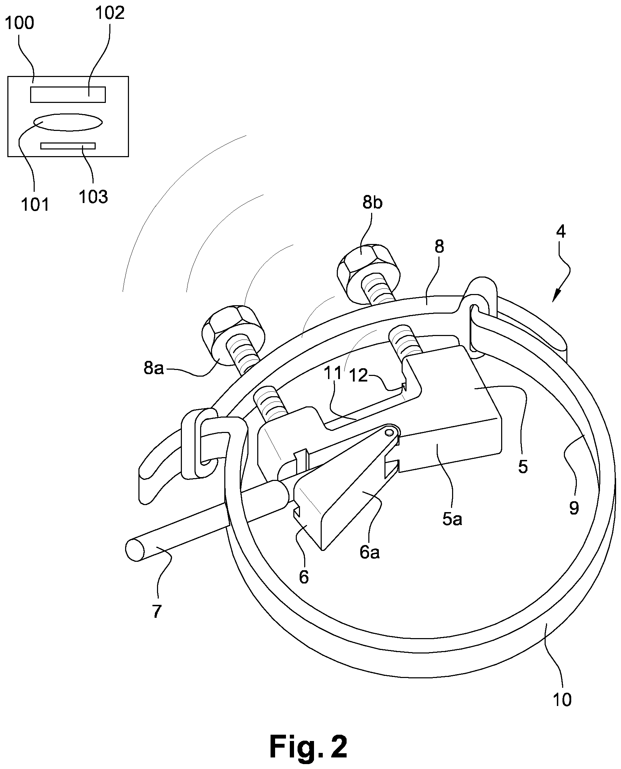

La figure 2 illustre un mode de réalisation particulier du premier organe de mesure 4.

Le premier organe de mesure4 comporte un cavalier 5 dont une partie de la face opposée 5a à deux bras du cavalier est destinée à venir reposer contre le tibia T. Le premier organe de mesure 4 comporte en outre un flan 6 qui est monté pivotant autour du cavalier 5 et dont une face 6a est destinée à venir reposer contre le tibia T. Une molette de réglage 7 permet de régler et de bloquer une orientation du flan mobile 6 par rapport au cavalier 5. A cet effet, par une liaison vis-écrou, la molette de réglage 7 entraîne en translation un doigt qui, engagé dans une rainure du flan 6, provoque le soulèvement ou l'abaissement du flan 6.

De plus, le premier organe de mesure 4 comporte un arceau 8 disposé à l'amont du cavalier 5 relativement au tibia T. Le premier organe de mesure 4 comporte également deux vis de réglage 8a, 8b qui coopèrent toutes deux avec l'arceau 8, chaque vis de réglage 8a, 8b coopérant en outre avec un des bras du cavalier 5.

Le premier organe de mesure 4 comporte ici deux sangles 9, 10 qui sont liées à chacune de leurs extrémités à des extrémités correspondantes de l'arceau 8.

Ainsi, pour disposer le premier organe de mesure 4 contre le tibia T d'un patient, on attache dans un premier temps le premier organe de mesure 4 à la partie crurale 1 associée au tibia T avec les sangles 9, 10. Le premier organe de mesure 4 est conformé pour qu'alors, les faces du cavalier 5a et du flan 6a reposent contre le tibia T. Puis, on règle la position du cavalier 5 et du flan 6 vis-à-vis du tibia T grâce aux vis de réglage 8a, 8b et à la molette de réglage 7 de sorte à ce que le premier organe de mesure 4 épouse le mieux possible la forme du tibia.

Selon l'invention, le premier organe de mesure 4 comporte des moyens pour relever une position absolue du tibia. Ici le premier organe de mesure 4 comporte un capteur de position 11 lié rigidement au cavalier 5 entre les deux bras dudit cavalier. Comme le capteur de position 101 est lié au cavalier 5 qui repose lui-même contre le tibia, le capteur de position 11 relève bien une position absolue du tibia T.

En outre, le premier organe de mesure 4 comporte des moyens 11 pour générer un signal représentatif de cette position. Par exemple, lesdits moyens comportent des moyens d'acquisition de la position absolue relevée par le capteur et des moyens de transmission à distance dudit signal à un organe de calcul distant 100. Par exemple, les moyens de transmission comportent un émetteur électromagnétique sans fil de type bluetooth.

Selon un mode de réalisation privilégié, le premier organe de mesure 4 comporte une source d'alimentation interne pour l'alimentation au moins des moyens pour relever une position absolue du tibia T et des moyens pour générer un signal représentatif de cette position. Par exemple, la source d'alimentation interne est une pile rechargeable.

Le premier organe de mesure 4 relève ainsi une position absolue du tibia T en tirant avantageusement profit d'un contact direct avec le tibia T. De plus, notamment grâce au flan 6 et au cavalier 5, le premier organe de mesure4 épouse au mieux la forme du tibia T de sorte que le signal généré soit très représentatif d'une position absolue du tibia T. Le premier organe de mesure 4 est en outre adaptable à différents patients et donc différentes morphologie.

En référence à la figure 1, le dispositif de détermination selon l'invention comporte en outre un deuxième organe 20, un troisième organe 30 et un quatrième organe 40 de mesure qui sont destinés à être positionnés relativement au fémur F. Chaque deuxième, troisième, quatrième organes de mesure 20, 30, 40 encerclent chacun la cuisse 2 associée au fémur F à des hauteurs différentes.

Ici, chaque deuxième, troisième, quatrième organe de mesure20, 30, 40 comporte une bande élastique 21, 31, 41 pour son attache autour de la cuisse 2, la bande élastique se fermant par exemple par une fixation de type Velcro® (fixation par boucles et crochets). Selon un mode de réalisation privilégié, chaque bande 21, 31, 41 comporte des poches P dans lesquelles est positionné l'organe de mesure associé.

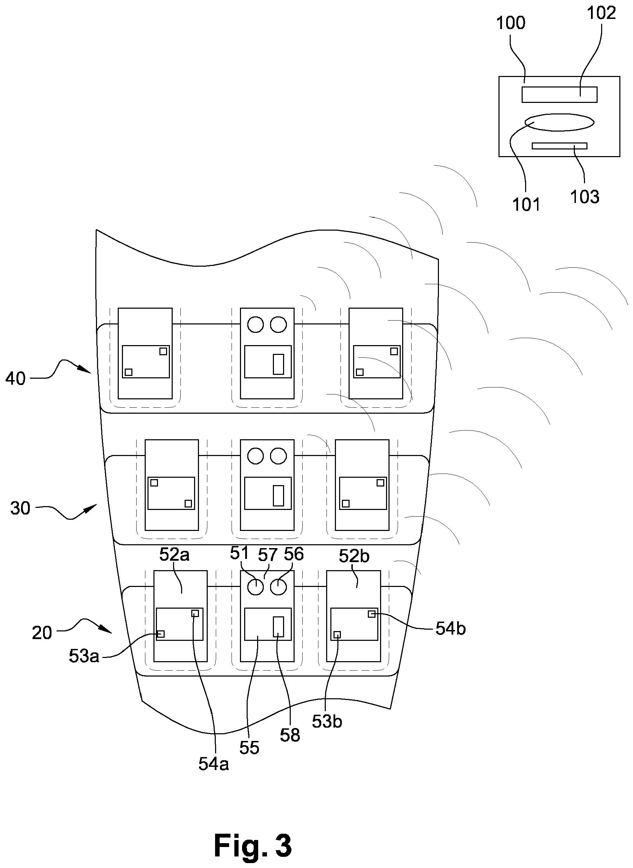

En référence à la figure 3, selon un mode de réalisation particulier, les deuxième, troisième et quatrième organes 20, 30, 40 sont tous identiques. Seul le deuxième organe de mesure 20 va être ici décrit mais il va de soi que cette description est également applicable au troisième 30 et quatrième organe de mesure 40. De façon privilégiée, les deuxième, troisième et quatrième organes fonctionnent tous de façon simultanée.

Le deuxième organe de mesure 20 comporte des moyens pour relever des données représentatives d'une position du fémur F.

Selon un mode de réalisation privilégié, lesdits moyens comportent un premier capteur de position 52 et un deuxième capteur de position 53, chaque capteur de position 52a, 52b comportant chacun deux accéléromètres respectivement 53a, 54a et 53b, 54b. Lesdits moyens comportent en outre des moyens d'acquisition 51 de données relevées par les capteurs 52a, 52b et des moyens de traitement 56 desdites données.

Ainsi, à partir de données d'accélérations brutes transmises par les accéléromètres 53a, 54a et 53b, 54b qui lui sont associés, chaque capteur 52a, 52b traduit lesdites données brutes en données de rotation absolue. Puis chaque capteur 52a, 52b intègre les données de rotation absolue pour en déduire des données de position. Ces données de position sont alors acquises par les moyens d'acquisition 51 qui communiquent avec les moyens de traitement 56 pour en déduire des données représentatives d'une position du fémur F.

Le deuxième organe de mesure 20 comporte également des moyens 55 pour générer un signal représentatif de ces données représentatives d'une position du fémur F. Lesdits moyens 55 de génération d'un signal comportant des moyens de transmission à distance 58 dudit signal à l'organe de calcul distant 100. Par exemple, les moyens de transmission comportent un émetteur électromagnétique sans fil de type bluetooth.

De préférence, le deuxième organe de mesure 20 comporte un boîtier 57 dans lequel sont disposés les moyens 55 de génération d'un signal, les moyens d'acquisition 51 et les moyens de traitement 58. Les capteurs 52a, 52b sont alors reliés audit boîtier 57 par exemple de façon filaire.

De préférence, les capteurs 52a, 52b sont insérés chacun dans une poche entourant une poche centrale dans laquelle est inséré le boîtier 57. Ainsi, le dispositif selon l'invention est conformé de sorte que les capteurs 52a, 52b soient disposés à des positions éloignées sur une même hauteur de cuisse 2.

Selon un mode de réalisation privilégié, le deuxième organe de mesure 20 comporte une source d'alimentation interne pour au moins alimenter les moyens pour relever des données représentatives d'une position du fémur F et les moyens pour générer un signal représentatif de ces données. Par exemple, la source d'alimentation interne est une pile rechargeable.

En référence aux figures 1, 2 et 3, l'organe de calcul 100 comporte des moyens de communication 101 pour recevoir les signaux générés par le premier, le deuxième, le troisième organe et quatrième organe de calcul 10, 20, 30, 40. Par exemple, l'organe de calcul 100 comporte un récepteur électromagnétique sans fil de type bluetooth.

L'organe de calcul comporte en outre des moyens de comparaison 102 pour confronter les signaux émis par le deuxième, troisième et quatrième organe de mesures 20, 30, 40 entre eux et en déduire une position absolue du fémur.

Grâce au croisement des signaux émis par le deuxième, troisième et quatrième organe de mesures 20, 30, 40 reçus par l'organe de calcul 100, le dispositif de l'invention permet de déterminer très correctement une position absolue du fémur.

L'organe de calcul comporte en outre des moyens de traitement 103 qui analyse le signal émis par le premier organe de mesure 100 et la position absolue du fémur F estimée par les moyens de comparaison 102. Les moyens de traitement 103 en déduisent alors une position relative du fémur F par rapport au tibia T.

Les moyens de traitement 103 et les moyens de comparaison sont bien entendu adaptés à mettre en forme les signaux reçus et notamment à filtrer des bruits desdits signaux.

De façon avantageuse, les deuxième, troisième et quatrième organes 20, 30, 40 sont de petites tailles et de faibles masses. Les inventeurs ont ainsi pu fabriquer des capteurs de position de 50 millimètres x 50 millimètres x 20 millimètres pour une masse de 45 grammes et de 50 millimètres x 50 millimètres x 15 millimètres pour une masse de 40 grammes. Les inventeurs ont également pu fabriquer des moyens pour générer un signal représentatif d'une position absolue du fémur de 70 millimètres x 140 millimètres x 25 millimètres et même de 50 millimètres x 50 millimètres x 15 millimètres.

Bien entendu l'invention n'est pas limitée au mode de réalisation décrit et on peut y apporter des variantes de réalisation sans sortir du cadre de l'invention tel que défini par les revendications.

Bien qu'ici les différents organes du dispositif transmettent les signaux par communication électromagnétique sans fil de type bluetooth, les différents organes pourront transmettre autrement les signaux par exemple par simple liaison filaire, par wifi...

De la même façon, les différents organes pourront comporter d'autres types de source d'alimentation interne comme par exemple une batterie. En outre, les différents organes pourront ne pas comporter de source d'alimentation interne mais être simplement alimentés de façon filaire à une source d'alimentation externe.

Le dispositif pourra comporter un nombre différent d'organe de mesure destinés à être positionnés sur le fémur que celui décrit à condition qu'il en comporte au moins deux.

Les organes de mesure destinés à être positionnés sur le fémur pourront être différents de ceux décrits à condition qu'ils comportent chacun des moyens pour relever des données représentatives d'une position d'un fémur et des moyens pour générer un signal représentatif de ces données. Par exemple, lesdits organes pourront ne pas tous être identiques. Lesdits organes pourront comporter des bandes élastiques sans poches pour leur attache à la cuisse, les moyens pour relever une position absolue du fémur et les moyens pour générer un signal représentatif de cette position étant fixés à la bande élastique par une attache de type colle, Velcro®... Chacun desdits organe pourra comporter un nombre de capteur de position différent de celui décrit. Un traitement des données fournies par les accéléromètres pourra être différent de celui décrit. Par exemple, le filtrage du signal généré pourra être directement effectué par les moyens de génération d'un signal.

## Revendications

1. Dispositif de détermination d'une position relative d'un fémur (F) par rapport à un tibia (T) comportant au moins :
- un premier organe de mesure (4) destiné à être positionné contre le tibia et comportant des moyens (12) pour générer un signal représentatif d'une position du premier organe de mesure ;
- un deuxième et un troisième organe de mesure (20 ; 30) destinés à être positionnés relativement au fémur en encerclant chacun la cuisse (2) associée au fémur à des hauteurs différentes, le deuxième et le troisième organe de mesure comportant chacun des moyens pour générer un signal représentatif d'une position de l'organe de mesure associé ;
- un organe de calcul (100) comportant des moyens de communication (101) pour recevoir au moins les signaux générés par le premier, deuxième et troisième organes de mesure, des moyens de comparaison (102) qui confrontent les signaux des deuxième et troisième organes pour en déduire une position absolue du fémur et des moyens de traitement (103) qui analysent le signal du premier organe de mesure et la position absolue du fémur pour en déduire une position relative du fémur par rapport au tibia.

2. Dispositif de détermination selon la revendication 1, dans lequel les moyens pour relever des données représentatives d'une position du fémur (F) comportent un premier capteur de position (52a) et un deuxième capteur de position (52b), chaque capteur comportant deux accéléromètres (53a, 54a, 53b, 54b).

3. Dispositif de détermination selon la revendication 2, conformé de sorte que les capteurs soient disposés à des positions éloignées sur une même hauteur de cuisse (2).

4. Dispositif de détermination selon l'une des revendications précédentes comportant en outre un quatrième organe de mesure (40) destiné à être positionné relativement au fémur (F) en encerclant la cuisse (2) associée au fémur à une hauteur différente du deuxième (20) et du troisième (30) organe de mesure, le quatrième organe comportant des moyens pour relever des données représentatives d'une position du fémur et des moyens pour générer un signal représentatif de ces données.

## Patentansprüche

1. Vorrichtung zum Bestimmen einer relativen Position eines Oberschenkelknochens (F) in Bezug zu einem Schienbein (T), die mindestens Folgendes umfasst:
- ein erstes Messorgan (4), das dazu bestimmt ist, gegen das Schienbein positioniert zu werden, und das Mittel (12) zum Erzeugen eines Signals umfasst, das für eine Position des ersten Messorgans repräsentativ ist;
- ein zweites und ein drittes Messorgan (20; 30), die dazu bestimmt sind, in Bezug zu dem Oberschenkelknochen positioniert zu sein, indem sie jeweils den Oberschenkel (2), der zu dem Oberschenkelknochen gehört, an unterschiedlichen Höhen einkreisen, wobei das zweite und das dritte Messorgan jeweils Mittel zum Erzeugen eines Signals umfassen, das für eine Position des dazugehörenden Messorgans repräsentativ ist;
- ein Rechenorgan (100), das Kommunikationsmittel (101) umfasst, um mindestens die Signale zu empfangen, die von dem ersten, zweiten und dritten Messorgan erzeugt werden, Vergleichsmittel (102), die die Signale des zweiten und dritten Organs einander gegenüberstellen, um daraus eine Absolutposition des Oberschenkelknochens abzuleiten, und Verarbeitungsmittel (103), die das Signal des ersten Messorgans und die Absolutposition des Oberschenkelknochens analysieren, um daraus eine relative Position des Oberschenkelknochens in Bezug zu dem Schienbein abzuleiten.

2. Vorrichtung zum Bestimmen nach Anspruch 1, wobei die Mittel zum Messen der Daten, die für eine Position des Oberschenkelknochens (F) repräsentativ sind, einen ersten Positionssensor (52a) und einen zweiten Positionssensor (52b) umfassen, wobei jeder Sensor zwei Beschleunigungsmessvorrichtungen (53a, 54a, 53b, 54b) umfasst.

3. Vorrichtung zum Bestimmen nach Anspruch 2, die derart gestaltet ist, dass die Sensoren an Positionen angeordnet sind, die auf ein und derselben Höhe des Oberschenkels (2) beabstandet sind.

4. Vorrichtung zum Bestimmen nach einem der vorhergehenden Ansprüche, die außerdem ein viertes Messorgan (40) umfasst, das dazu bestimmt ist, in Bezug zu dem Oberschenkelknochen (F) positioniert zu sein indem es den Oberschenkel (2), der zu dem Oberschenkelknochen gehört, in einer Höhe einkreist, die von dem zweiten (20) und dritten (30) Messorgan unterschiedlich ist, wobei das vierte Organ Mittel umfasst, um Daten zu erheben, die für eine Position des Oberschenkelknochens repräsentativ sind, und Mittel zum Erzeugen eines Signals, das für diese Daten repräsentativ ist.

## Claims

1. A device for determining a relative position of a femur (F) relative to a tibia (T) including at least:
- a first measuring member (4) intended to be positioned against the tibia and including means (12) for generating a signal representative of a position of the first measuring member;
- a second and a third measuring member (20; 30) intended to be positioned relative to the femur by encircling each the thigh (2) associated with the femur at different heights, the second and third measuring members each including means for generating a signal representative of a position of the associated measuring member;
- a calculating member (100) including communication means (101) for receiving at least the signals generated by the first, second and third measuring members, comparison means (102) which confront the signals of the second and third members in order to deduce therefrom an absolute position of the femur and processing means (103) which analyze the signal of the first measuring member and the absolute position of the femur in order to deduce therefrom a relative position of the femur relative to the tibia.

2. The determination device according to claim 1, wherein the means for collecting data representative of a position of the femur (F) include a first position sensor (52a) and a second position sensor (52b), each sensor including two accelerometers (53a, 54a, 53b, 54b).

3. The determination device according to claim 2, shaped so that the sensors are disposed at spaced positions on the same thigh height (2).

4. The determination device according to any of the preceding claims, further including a fourth measuring member (40) intended to be positioned relative to the femur (F) by encircling the thigh (2) associated with the femur at a different height from the second (20) and third (30) measuring members, the fourth member including means for collecting data representative of a position of the femur and means for generating a signal representative of said data.
